(19) Europäisches Patentamt
European Patent Office
Office européen des brevets

(11) **EP 1 430 437 B1**

(12) **EUROPÄISCHE PATENTSCHRIFT**

(45) Veröffentlichungstag und Bekanntmachung des Hinweises auf die Patenterteilung:
**27.05.2009 Patentblatt 2009/22**

(21) Anmeldenummer: **02798646.2**

(22) Anmeldetag: **12.09.2002**

(51) Int Cl.:
**G06F 19/00** (2006.01)

(86) Internationale Anmeldenummer:
**PCT/EP2002/010235**

(87) Internationale Veröffentlichungsnummer:
**WO 2003/025219 (27.03.2003 Gazette 2003/13)**

(54) **VERFAHREN ZUR SEQUENZIERUNG VON NUKLEINSÄUREN**

METHOD FOR SEQUENCING NUCLEIC ACIDS

PROCEDE POUR SEQUENCER DES ACIDES NUCLEIQUES

(84) Benannte Vertragsstaaten:
**AT BE BG CH CY CZ DE DK EE ES FI FR GB GR IE IT LI LU MC NL PT SE SK TR**

(30) Priorität: **14.09.2001 AT 14442001**

(43) Veröffentlichungstag der Anmeldung:
**23.06.2004 Patentblatt 2004/26**

(73) Patentinhaber:
• **Roche Diagnostics GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AT BE BG CH CY CZ DK EE ES FI FR GB GR IE IT LI LU MC NL PT SE SK TR**

(72) Erfinder:
• **HUBER, Christian**
**66117 Saarbrücken (DE)**
• **OBERACHER, Herbert**
**66125 Saarbrücken (DE)**

(56) Entgegenhaltungen:
**US-A- 6 017 693**

• **NI J, POMERANTZ S, ROZENSKI J, ZHANG Y, MCCLOSKEY J: "Interpretation of Oligonucleotide Mass Spectra for Determination of Sequence Using Electrospray Ionization and Tandem Mass Spectrometry" ANALYTICAL CHEMISTRY, Bd. 68, Nr. 13, 1. Juli 1996 (1996-07-01), Seiten 1989-1999, XP002263308 in der Anmeldung erwähnt**
• **OBERACHER H, WELLENZOHN B, HUBER C: "Comparative Sequencing of Nucleic Acids by Liquid Chromatography - Tandem Mass Spectrometry" ANALYTICAL CHEMISTRY, Bd. 74, Nr. 1, 1. Januar 2002 (2002-01-01), Seiten 211-218, XP002263309**
• **ROZENSKI J, MCCLOSKEY J: "SOS: A Simple Interactive Program for ab initio Oligonucleotide Sequencing by Mass Spectrometry" AMERICAN SOCIETY FOR MASS SPECTROMETRY, Bd. 13, Nr. 3, 1. März 2002 (2002-03-01), Seiten 200-203, XP002263310**
• **LITTLE DP, AASERUD DJ, VALASKOVIC GA, MCLAFFERTY FW: "Sequence Information from 42-108-mer DNAs (Complete for a 50-mer) by Tandem Mass Spectrometry" JOURNAL OF THE AMERICAN CHEMICAL SOCIETY, [Online] Bd. 118, 1996, Seiten 9352-9359, XP002263311**

## Beschreibung

Die Erfindung bezieht sich auf ein Verfahren zur Sequenzierung von Nukleinsäuren, wobei die für eine angenommene Sequenz berechneten Werte des CID-Spektrums mit dem gemessenen Spektrum der Nukleinsäure verglichen und der Grad der Übereinstimmung berechnet wird.

Bei derartigen Verfahren werden zunächst Nukleinsäuren ionisiert, in einem ersten Spektrometer selektiert und dann durch Kollision mit gasförmigen Atomen oder Molekülen fragmentiert (als collision induced dissociation (CID) bezeichnet). Die Fragmentierung erfolgt nach einer bekannten Gesetzmäßigkeit, weshalb die massenspektrographische Untersuchung der Fragmente einen Rückschluß auf das Ausgangsion erlaubt.

Bei der Fragmentierung der Nukleinsäuren entstehen am 5'-Ende der Ionen (a-B Ionen), am 3'-Ende (w Ionen). In Ni, J.; Pomerantz, S. C.; Rozenski, J.; Zhang, Y.; McCloskey J.A. Anal. Chem. 1996, 68, 1989-99, ist ein Verfahren angegeben, wie man von den Enden ausgehend die gesamte Sequenz von Grund auf erschließen kann. Da zunehmend unüberwindbare Fehlstellen auftreten, ist dieses Verfahren auf Nukleinsäuren geringer Länge (höchstens 15-mer) beschränkt. In US-PS 6,017,693 (Yates et al) ist andererseits ein Verfahren der eingangs definierten Art beschrieben, bei welchem eine Bibliothek von Peptiden daraufhin überprüft wird, ob sie eine dem gemessenen Spektrum entsprechende Verbindung enthält. In dieser Veröffentlichung ist auch die Ausdehnung des Verfahrens auf Oligonukleotide angeregt, doch ist das Verfahren wegen der riesigen Zahl zu überprüfender Ausgangswerte typischerweise auf 10-mere und darunter beschränkt.

Der Erfindung liegt die Aufgabe zugrunde, die bekannten Verfahren für deutlich längere Sequenzen anwendbar zu machen. Im wesentlichen wird dies erreicht, indem primär nicht die Frage nach der korrekten Sequenz gestellt wird, sondern die Frage, ob durch systematische Veränderung einer Ausgangssequenz eine Verbesserung der Übereinstimmung mit dem gemessenen Spektrum erzielt werden kann.

Die Erfindung wird durch die Merkmale des Anspruchs 1 definiert.

Grundsätzlich kann mit der Erfindung ausgehend von einer zufällig günstig angenommenen Ausgangssequenz durch wiederholte Anwendung des Verfahrens eine zunehmende Annäherung an die korrekte Sequenz erzielt werden. Seine eigentliche Stärke hat das Verfahren jedoch bei der Feststellung und Lokalisierung von Punktmutationen sowie von Weglassungen oder Einfügungen von Nukleinsäuren-Monomeren in eine bekannte Sequenz. Besonders dann, wenn die Zahl der Nukleotide bekannt ist, ist es vorteilhaft, wenn an einer bestimmten Stelle der angenommenen Sequenz jedes der vier möglichen Nukleotide eingesetzt und jeweils der Grad der Übereinstimmung mit dem Spektrum berechnet wird. Führt man dieses Verfahren für jede Stelle der Sequenz durch und setzt jeweils das Nukleotid mit der besten Übereinstimmung in die Sequenz ein, so ergibt sich schließlich für die gesamte Sequenz optimale Übereinstimmung.

Zur Berechnung der Übereinstimmung von berechnetem und gemessenem Spektrum wird ein Algorithmus verwendet, welcher auf der Feststellung beruht, ob an der berechneten Stelle im gemessenen Spektrum ein Meßwert vorliegt oder nicht.

Die Werte von m/z für ein bestimmtes Fragment sind mit großer Genauigkeit zu berechnen, während sich die Intensität der Messwerte, also die Häufigkeitsverteilung der verschiedenen Fragmente, schwer voraussagen lässt. Davon ausgehend ist es zunächst sinnvoll, von einer Prognose der Intensitäten überhaupt abzusehen, das Eintreffen eines vorausgesagten Messwertes im gemessenen Spektrum aber umso höher zu bewerten, je größer die gemessene Intensität ist. Da die Werte von m/z mit größerer Genauigkeit zu berechnen als zu messen sind, ist andererseits ein Differenzwert festzulegen, bis zu dem man die Voraussage als erfüllt ansieht. Diese Festlegung erfolgt durch die Forderung, dass die Zahl der falschen Treffer und der falschen Fehlanzeigen minimiert werden soll.

Weitere Einzelheiten der Erfindung werden anschließend anhand von Diagrammen und Ausführungsbeispielen diskutiert.

Fig. 1 erläutert die Berechnung eines Fragmentionenspektrums,
Fig. 2 betrifft die Vorgangsweise beim Vergleich eines berechneten und eines gemessenen Spektrums,
Fig. 3 bezieht sich auf die Optimierung des Algorithmus von Fig. 2,
Fig. 4 zeigt die relative Bedeutung der Faktoren des Algorithmus von Fig. 2,
Fig. 5 ist das gemessene Spektrum eines 20-mer Oligodesoxynukleotides,
Fig. 6a und 6b zeigen die Ableitung einer Sequenz maximaler Übereinstimmung mit Fig. 5,
Fig. 7 betrifft die Sequenzierung eines 51-mer Oligodesoxynukleotides,
Fig. 8 betrifft die Überprüfung der Sequenz eines 80-mer Oligodesoxynukleotides.

## Prinzip der Sequenzierung mittels Tandem-Massenspektrometrie

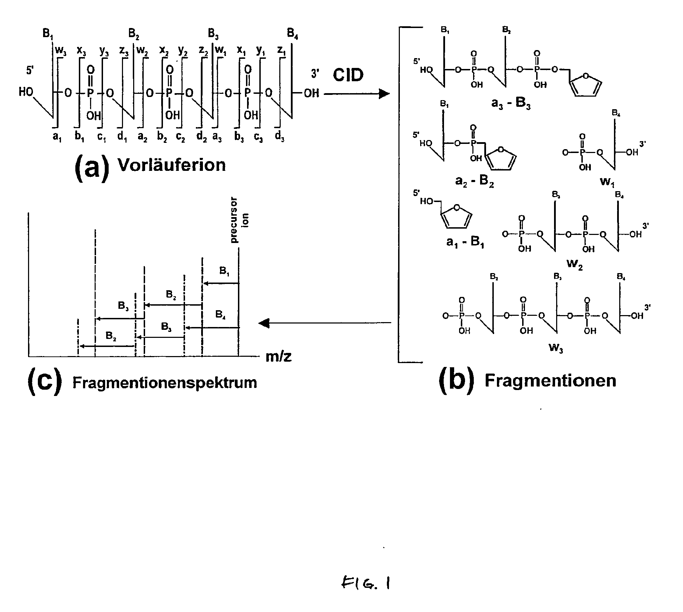

Durch kollisionsiduzierte Fragmentierung werden Nukleinsäuren in einer sequenzspezifischen Weise gespalten, wodurch Bruchstücke des Vorläuferions entstehen, die entsprechend der Art des Bindungsbruches in der Phosphodiester-Gruppe und des Ladungsverbleibes bezeichnet werden. Während geladene Fragmente mit einem intakten 5'-terminalen Ende als a, b, c, d - Fragmente bezeichnet werden, werden geladene Fragmente mit einem intakten 3'-terminalen Ende als w, x, y, z - Fragmente bezeichnet (Fig. 1a). Die Anzahl der Desoxyribosereste eines Fragments wird mit tiefgestellten Zahlen und der Ladungszustand mit hochgestellten Zahlen angegeben. So steht $(a_5)^{2-}$ für ein Fragment der a-Serie, das aus fünf Nukleotideinheiten besteht und zweifach negativ geladen ist. Fragmente mit einer zusätzlichen Abspaltung einer Base werden durch die Zufügung von -B, z. B. als $(a_n-B_n)^{m-}$ bezeichnet.

Obwohl die Fragmentierung grundsätzlich an jeder Bindung stattfinden kann, zeigte sich, dass es einen Hauptfragmentierungsweg für Oligonukleotidanionen gibt. Dabei kommt es zuerst zu einem Bruch der 3'-C-O-Bindung des Zuckers, was anschließend zu einem Verlust der Base und Bildung von w- und dazu komplementären (a-B)-Ionen führt (Fig. 1b). Fragmentionen eines bestimmten Typs gehören zu einer bestimmten Ionenserie, wobei die Massendifferenzen innerhalb einer Ionenserie von den Massen der Basen in der Sequenz bestimmt werden. Daher lässt sich anhand der Massendifferenzen in Ionenserien die Sequenz einer fragmentierten Nukleinsäure ablesen (Fig. 1c).

## Strategie des vergleichenden Sequenzierens

Ausgangspunkt des erfindungsgemäßen Sequenzierungsansatzes ist die Tatsache, dass eine vollständige Neubestimmung der Sequenz für viele genomspezifische Anwendungen gar nicht notwendig ist. Meist genügt es, eine Sequenz zu verifizieren bzw. geringe Abweichungen zu einer bekannten Sequenz zu finden. Dabei stellt die Sequenzierung mittels MS/MS eine attraktive Alternative zur herkömmlich verwendeten, von Sanger entwickelten Sequenzierungsmethode dar. Der Grund hierfür ist, dass sequenzspezifische Daten innerhalb von Sekunden durch kollisionsinduzierte Fragmentierung und nachfolgende Analyse der Fragmentionen erhalten werden können.

Die Güte der Übereinstimmung zwischen einem gemessenen MS/MS-Spektrum und einem Satz von Fragmentionen, die ausgehend von einer angenommenen Referenz berechnet werden, kann durch einen praktisch beliebigen mathematischen Algorithmus beschrieben werden. Als Beispiel wird ein Algorithmus erläutert, in dem die Übereinstimmung zwischen gemessenem Spektrum und den berechneten Fragmentionen durch den Wert der Fitness (FS) gekennzeichnet ist. In die Fitness fließen der absolute Unterschied $\Delta$ zwischen den gemessenen und den berechneten m/z-Werten, die relative Intensität I% der Fragmentionen, die Zahl der zugeordneten Fragmente K und die Zahl der nicht mit einem Fragmention abgedeckten Nukleotidpositionen M ein. Je kleiner der Wert der Fitness umso größer ist die Übereinstimmung des gemessenen Spektrums mit den berechneten m/z-Werten.

Um jene Sequenz zu finden, die am besten mit den experimentellen Daten eines MS/MS Spektrums übereinstimmt, wird die erste Referenzsequenz sequenziell variiert, indem an jeder Position der Sequenz alle vier möglichen Nukleotide A, T, G und C eingebaut werden. Jene Sequenz, deren Wert für die Fitness am geringsten ist, wird dann als die richtige identifiziert.

## Entwicklung des Sequenzierungsalgorithmus

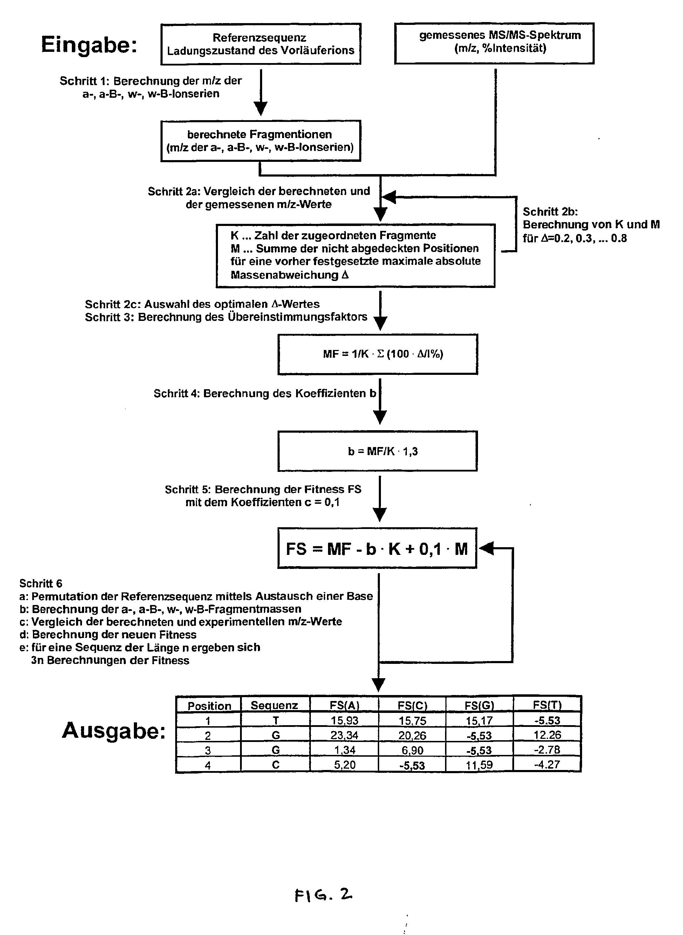

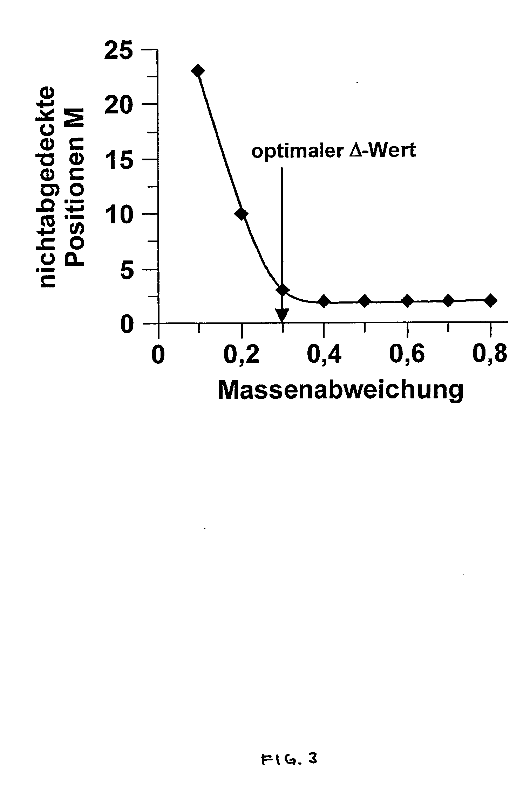

In Fig. 2 ist der Ablauf für das vergleichende Sequenzieren von Nukleinsäuren dargestellt. Die Eingabeparameter sind einerseits die Referenzsequenz zusammen mit dem Ladungszustand des Vorläuferions und andererseits eine Liste von experimentell bestimmten m/z-Werten und deren relative Intensitäten I%. Ausgehend von der Referenzsequenz wird eine Liste von monoisotopischen m/z-Werten für die a-, a-B-, w- und w-B-Ionenserien berechnet, wobei alle möglichen Ladungszustände der Fragmentionen von 1- bis zum Ladungszustand des Vorläuferions berücksichtigt werden (Schritt 1, Fig. 2). Anschließend werden die berechneten und experimentell gefundenen m/z-Werte miteinander verglichen (Schritt 2a, Fig. 2). Das Ergebnis dieses Vergleichs ist die Zahl der Fragmentionen (K), denen ein berechneter m/z-Wert zugeordnet werden konnte, und die Summe an nicht abgedeckten a- bzw. w-Positionen (M). Als nicht abgedeckte a-Postionen werden jene Positionen bezeichnet, für die weder ein a-B noch ein a-Fragment irgendeines möglichen Ladungszustandes im Spektrum gefunden werden konnte. Ebenso gilt für die nicht abgedeckten w-Postionen, dass weder ein w-B noch ein w-Fragment irgendeines möglichen Ladungszustandes im Spektrum zugeordnet werden konnte. Damit ein Fragment zugeordnet wird, muss der Absolutwert des Unterschieds zwischen dem gemessenen und dem berechneten m/z-Wert kleiner bzw. gleich einem Toleranzwert ($\Delta$) sein. Die Größen von K und M werden für alle $\Delta$-Werte zwischen 0.2 und 0.8 in 0.1 Schritten ansteigend berechnet (Schritt 2b, Fig. 2). Anschließend wird der optimale $\Delta$-Wert unter dem Kriterium ausgewählt, dass die Zahl der falsch positiven ($\Delta$ zu groß) bzw. der falsch negativen ($\Delta$ zu klein) Zuordnungen minimiert wird (Schritt 2c, Fig. 2). Der optimale $\Delta$-Wert kann aus einer Auftragung von M gegen $\Delta$ als jener $\Delta$-wert abgeleitet werden, bei dem die Kurve gerade den konstanten Wert erreicht (Fig. 3).

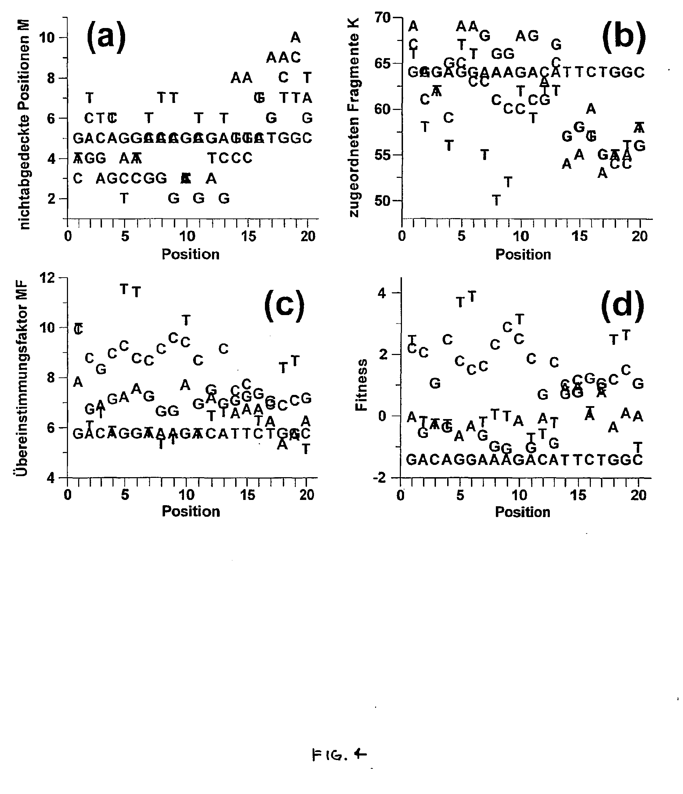

Der nächste Schritt des Algorithmus ist die Berechnung eines Übereinstimmungsfaktors MF, der die Qualität der Zuordnung der m/z-Werte in bezug auf Massenabweichung und Intensität widerspiegelt (Schritt 3, Fig. 2). Der Übereinstimmungsfaktor MF ist definiert als die Summe aller Quotienten der absoluten Massenabweichung $\Delta$ und der Intensität I (da die Intensität in % eingegeben wird, wird der Wert mit 100 multipliziert), die über die Zahl der Zuordnungen K gemittelt wird. Daraus ergibt sich folgende Formel $MF = 1/K \cdot \Sigma (100 \Delta/I\%)$ (Fig. 2). Die drei Parameter, der Übereinstimmungsfaktor MF, die Zahl der zugeordneten Fragmentionen K und die Summe an nichtabgedeckten Positionen M, werden mittels folgender Gleichung FS = a · MF → b · K + c · M zusammengefasst, um die Fitness der Sequenz zu beschreiben. Je kleiner die Massenabweichungen, je höher die Signalintensitäten, je größer die Zahl an Zuordnungen und je geringer die Summe an nichtabgedeckten Positionen ist, umso kleiner wird der Wert der Fitness. Wie Fig. 4 deutlich zeigt, kann nur durch das Zusammenspiel der drei Einzelteile der Formel (Übereinstimmungsfaktor MF, Zahl der zugeordneten Fragmentionen K und Summe an nichtabgedeckten Positionen M) die Sequenz korrekt verifiziert werden (Fig. 4d). Dagegen würde man dem MS/MS-Spektrum unter alleiniger Berücksichtigung der drei Einzelteile jeweils andere Sequenzen als besser passend zuordnen (Fig. 4a-c). Für das Zusammenfassen des Übereinstimmungsfaktors MF mit der Zahl der zugeordneten Fragmentionen K und der Summe an nichtabgedeckten Positionen M werden drei Koeffizienten a, b und c eingeführt, welche die Teile der Formel gewichten. Der Koeffizient a wird per Definition auf den Wert 1 festgesetzt, wogegen b und c empirisch bestimmt werden. Dabei wird der Koeffizient b so ausgewählt, dass das Verhältnis zwischen dem ersten und dem zweiten Teil der Formel, MF:(b·K), gleich 1:1,3 ist. Daher gilt b = MF/K · 1,3 (Schritt 4, Fig. 2). Ein Wert von 0,1 für den Koeffizienten c wurde als passend für alle hier angeführten Beispiele gefunden. Setzt man die Koeffizienten in obige Gleichung ein, erhält man für FS (Schritt 5, Fig. 2) :

$$FS = 1/K \cdot \sum \left( 100 \cdot \frac{\Delta}{I\%} \right) - b \cdot K + 0,1 \cdot M$$

Der primär berechnete Wert der Fitness ist ein Maß für die Übereinstimmung des gemessenen MS/MS-Spektrums mit dem von der Referenzsequenz ausgehend berechneten Satz an Fragmentionen. Um nun eventuell eine besser übereinstimmende Sequenz zu finden, oder die Identität von gemessener Sequenz mit der Referenzsequenz zu bestätigen, wird die Referenzsequenz systematisch an jeder Position variiert und anschließend für jede dieser mutierten Sequenzen eine neue Fitness berechnet. Dazu wird sequentiell an jeder Position die Base durch die drei anderen Basen ersetzt, während der Rest der Sequenz konstant gehalten wird (Schritt 6a, Fig. 2). Unter der Annahme, dass die Sequenz nur eine Mutation enthält, werden 3n Berechnungen der FS für eine Sequenz aus n Nukleotiden durchgeführt (Schritt 6b-e, Fig. 2). Hierbei ist es wichtig festzustellen, dass der Gewichtungsfaktor b für alle variierten Sequenzen konstant gehalten wird und dem im Schritt 4 des Algorithmus aus der Zahl der zugeordneten Fragmentionen (K) und der Summe an nichtabgedeckten Positionen (M) der ursprünglichen Referenzsequenz berechneten Wert (b = MF/K 1,3) entspricht. Das Ergebnis der gesamten Berechnung ist eine Matrix, welche die Fitness der Referenzsequenz (fettgeschriebene Nummern, Fig. 2) und der variierten Sequenzen an den verschiedenen Positionen der Sequenz enthält (Fig. 2). Zum Beispiel repräsentiert der FS(T)-Wert in Zeile 1 der Matrix (-5,53) die Fitness der Sequenz TGGC. Dagegen ist FS(A) der Wert für jene Sequenz, bei der das T an Position 1 durch ein A ausgetauscht wurde (15,93). Wie man an diesem Beispiel sehen kann, wurde der kleinste FS-Wert für die Referenzsequenz erhalten, was zum Schluss führt, das hier die beste Übereinstimmung zwischen dem experimentellen Spektrum und der Referenzsequenz besteht. Der Austausch einer Base würde durch einen FS-Wert angezeigt, der für eine mutierte Sequenz kleiner als der FS-Wert der Referenzsequenz ist.

**1. Beispiel: Vergleichendes Sequenzieren eines 20-mer Oligodesoxynukleotides**

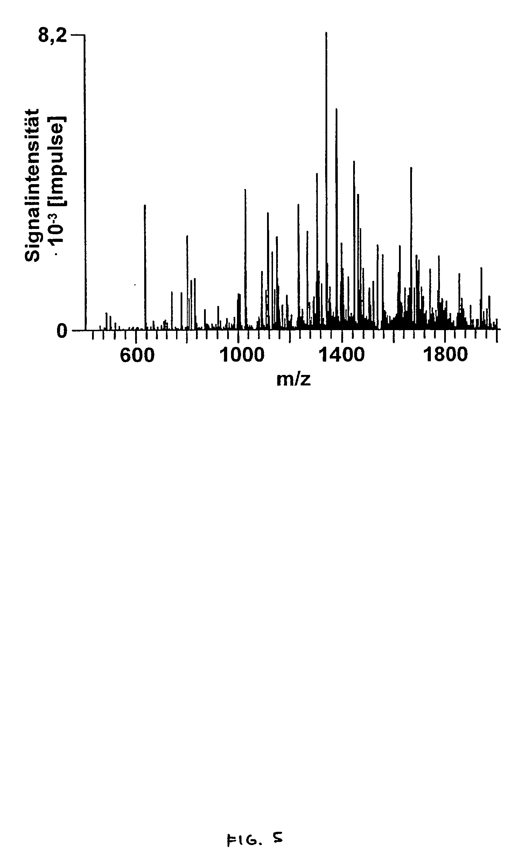

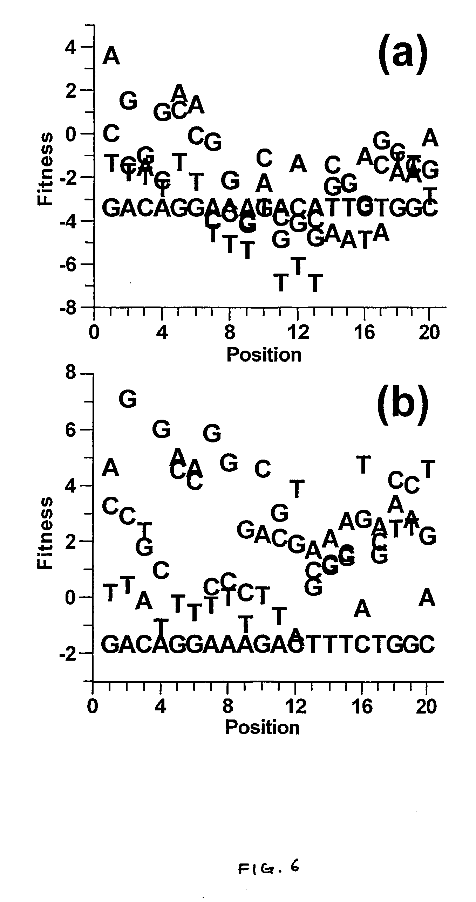

In Fig. 5 ist das MS/MS-Spektrum eines 20-mers der Sequenz GACAGGAAAG ACTTTCTGGC (Seq. ID No.: 1) dargestellt. Das Experiment wurde unter folgenden Bedingungen durchgeführt: Säule: Poly-(Styrol/Divinylbenzol)-Monolith, 60 × 0,2 mm I.D.; mobile Phase: (A) 25 mM TEAB, pH 8,4, (B) 25 mM TEAB, pH 8,4, 20 % Acetonitril; linearer Gradient: 5-50 % B in 10 min.; Flussrate: 3,0 μl/min; Temperatur: 50 °C; Scan: 420-2000 amu; Elektrospray-Spannung: 3,40 kV; Sheath-Gas: 40 Einheiten; Sheath-Flüssigkeit: Acetonitril, 3,0 μl/min; Produktionen von m/z 1542,5 (Ladungszustand: 4-); 4.0 amu Isolationsbreite; 35 % relative Kollisionsenergie; Probe: 222 ng des 20-mers. Die so erhaltenen massenspektroskopischen Daten (m/z, I%) fungierten dann als Eingabe für den vergleichenden Sequenzieralgorithmus. Als Referenzsequenz wurde die Sequenz GACAGGAAAGAC**A**TTCTGGC (Seq. ID No.: 2) verwendet, die an Position 13 ein A statt eines T enthielt. Tabelle 1 fasst die für diese Referenzsequenz anhand der oben beschriebenen Fragmentierungsmechanismen berechneten m/z Werte der a-, a-B-, w-und w-B Fragmentionen für die Ladungszustände von 1- bis 4- zusammen. Die Matrix der FS-Werte wurde dann entsprechend den Angaben im vorangegangenen Teil berechnet. Um das Ergebnis visuell leichter erfassen zu können, wurde die Matrix in Form eines Diagramms dargestellt, in dem die FS-Werte der einzelnen Basen, repräsentiert durch die Buchstaben A, T, G und C, gegen die Position in der Oligodesoxynukleotidsequenz aufgetragen wurden. Man erkennt in Fig. 6a, dass die Referenzsequenz für die ersten 6 Basen die beste Übereinstimmung lieferte, dass jedoch ab Position 7 die FS-Werte für andere Basen besser passten. Besonders der Austausch von T gegen A führte zu signifikant kleineren Werten für die Fitness, was eine Mutation von A nach T sehr nahe legte. Die genaue Position ergab sich aus dem Minimum der FS-Werte, wodurch die Mutation von A nach T an Position 13 eindeutig nachgewiesen wurde. Die so erhaltene Basensequenz (GACAGGAAAGACTTTCTGGC) (Seq. ID No.: 1) stimmt mit der theoretischen Basensequenz (GACAGGAAAGACTTTCTGGC) (Seq. ID No.: 1) vollständig überein. Wird nun im Algorithmus diese Sequenz als Referenzsequenz zusammen mit den massenspektroskopischen Daten aus Fig. 5 verwendet, ergibt sich Fig. 6b. Wie erwartet erhält man für die Referenzsequenz den kleinsten FS-Wert über die gesamte Sequenz. Augenscheinlich bestätigt das Diagramm die Identität der Referenzsequenz und damit die Sequenz des untersuchten Oligodesoxynukleotides. Aus diesen beiden Beispielen ergibt sich, dass man mit dieser Methode sowohl die Möglichkeit besitzt, die Identität einer unbekannten Sequenz mit einer Referenzsequenz zu überprüfen, als auch Punktmutationen in Vergleich zu einer bekannten Referenzsequenz zu detektieren.

## 2. Beispiel: Sequenzierung eines 51-mer Oligodesoxynukleotides

Ein interessantes Anwendungsgebiet der vergleichenden Sequenzierung ist die Detektion von Sequenzvariationen in genomischen DNA-Segmenten, die mittels Polymerase-Kettenreaktion (PCR) amplifiziert wurden. Diese Amplifikationsreaktion besteht aus drei sich zyklisch wiederholenden Schritten, bei denen nach Denaturierung der DNA Doppelhelix in Einzelstränge zwei verschiedene Oligodesoxynukleotide einer bestimmten Sequenz, sogenannte Primer, an spezifische, komplementäre Stellen hybridisiert werden, um anschließend in einer enzymkatalysierten Polymerisationsreaktion wieder zu den Doppelsträngen ergänzt zu werden. Das zu amplifizierende Segment der genomischen DNA wird durch die beiden Primer eingegrenzt. Da die PCR-Primer normalerweise eine Länge von ca. 20 Desoxynukleotiden aufweisen, muss ein amplifiziertes PCR-Produkt eine Länge von mehr als 40 Basenpaaren aufweisen, um relevante Sequenzinformation zu enthalten.

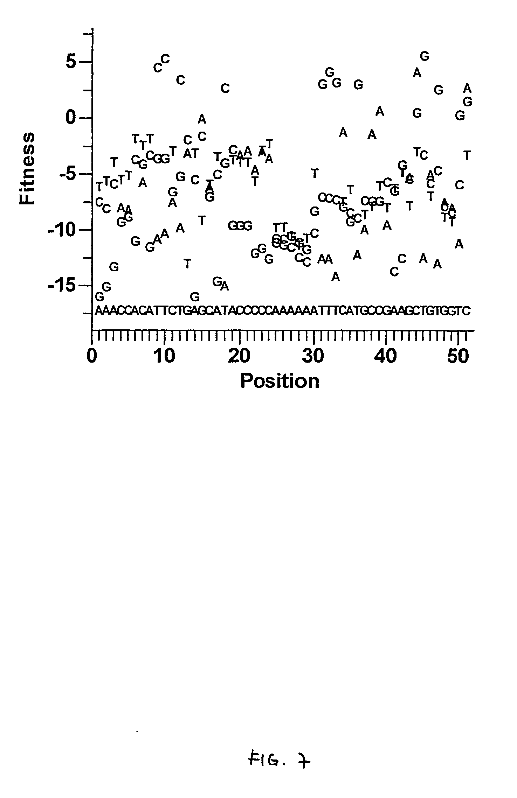

Wir wählten daher ein 51-mer, um die Möglichkeit der Identitätsüberprüfung für relativ lange DNA-Moleküle zu zeigen. Dazu wurde ein 51-mer (molekulare Masse: 15580,0) der Sequenz AAAACCACATT CTGAGCATAC CCC-CAAAAAA TTTCATGCCG AAGCTGTGGT C (Seq. ID No.: 3) fragmentiert und vermessen. Das Experiment wurde unter folgenden Bedingungen durchgeführt: Säule: Poly-(Styrol/Divinylbenzol)-Monolith, 60 × 0,2 mm I.D.; mobile Phase: (A) 25 mM Butyldimethylammoniumbicarbonat, pH 8,4, (B) 25 mM Butyldimethylammoniumbicarbonat, pH 8,4, 40 % Acetonitril; linearer Gradient: 5-70 % B in 10 min.; Flussrate: 2,0 $\mu$l/min; Temperatur: 50 °C; Scan: 470-2000 amu; Elektrospray-Spannung: 3,40 kV; Sheath-Gas: 40 Einheiten; Sheath-Flüssigkeit: Acetonitril, 3,0 $\mu$l/min; Produktionen von m/z 1729,7 (Ladungzustand: 9-); 4,0 amu Isolationsbreite; 17 % relative Kollisionsenergie; Probe: 635 ng des 51-mers. Das gemessene MS/MS-Spektrum wurde mit der Referenzsequenz verglichen und anhand der Fitness-Werte eindeutig als richtig identifiziert (Fig. 7).

## 3. Beispiel: Überprüfung der Sequenz eines 80-mer Oligodesoxynukleotides

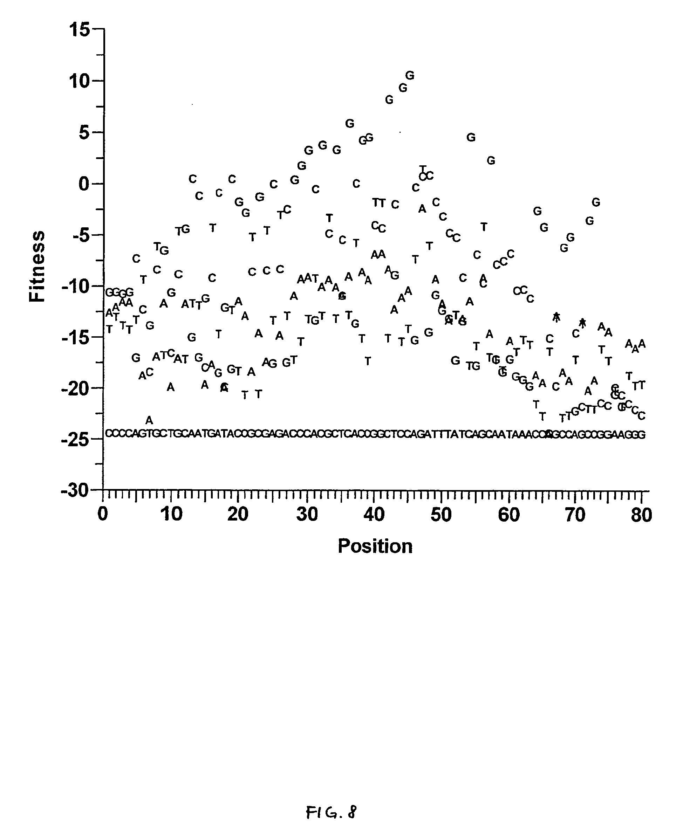

Ein weiteres Beispiel, das die Anwendbarkeit des Algorithmus zur Identifizierung von langen Oligodesoxynukleotiden zeigen soll, ist in Fig. 8 dargestellt. Hier wurde die Richtigkeit der Sequenz eines 80-mers durch Vergleich mit dem MS/MS-Spektrum überprüft (CCCCAGTGCT GCAATGATAC CGCGAGACCC ACGCTCACCG GCTCCAGATT TATCAGCAAT AAAACCAGCCA GCCGGAAGGG) (Seq. ID No.: 4). Das Experiment wurde unter folgenden Bedingungen durchgeführt: Säule: Poly-(Styrol/Divinylbenzol)-Monolith, 60 × 0,2 mm I.D.; mobile Phase: (A) 25 mM Butyldimethylammoniumbicarbonat, pH 8,4, (B) 25 mM Butyldimethylammoniumbicarbonat, pH 8,4, 40 % Acetonitril; linearer Gradient: 10-70 % B in 10 min.; Flussrate: 2,0 $\mu$l/min; Temperatur: 70°C; Scan: 470-2000 amu; Elektrospray-Spannung: 5,0 kV; Sheath-Gas: 100 Einheiten; Sheath-Flüssigkeit: Acetonitril, 3,0 $\mu$l/min; Produktionen von m/z 1065,4 (Ladungszustand: 23-); 4,0 amu Isolationsbreite; 17 % relative Kollisionsenergie; Probe: 478 ng des 80-mers. Es zeigte sich, dass der FS-Wert für die Referenzsequenz im Vergleich zu allen mutierten Sequenzen immer den kleinsten Wert besaß. Damit konnte die Identität der angenommenen Sequenz eindeutig nachgewiesen werden (Fig. 8).

Tabelle 1. Berechnete m/z Werte der Ladungszustände 1- bis 4- der a-, a-B-, w- und w-B-Fragmentionen der Referenzsequenz GACAGGAAAGACATTCTGGC (Seq. ID No.: 2).

| Typ | Ladungszustand | | | | Typ | Ladungszustand | | | |
|---|---|---|---|---|---|---|---|---|---|
| | -1 | -2 | -3 | -4 | | -1 | -2 | -3 | -4 |
| a1-B1 | 97.04 | 48.02 | 31.68 | 23.51 | w11-B10 | 3258.51 | 1628.76 | 1085.50 | 813.88 |
| w1-B20 | 195.01 | 97.01 | 64.34 | 48.00 | a11-B11 | 3267.58 | 1633.29 | 1088.53 | 816.15 |
| a1 | 248.09 | 123.55 | 82.03 | 61.27 | a11 | 3402.63 | 1700.82 | 1133.54 | 849.91 |
| w1 | 306.06 | 152.53 | 101.35 | 75.77 | w11 | 3409.56 | 1704.28 | 1135.85 | 851.64 |
| a2-B2 | 426.09 | 212.55 | 141.36 | 105.77 | a12-B12 | 3580.64 | 1789.82 | 1192.88 | 894.41 |
| w2-B19 | 484.06 | 241.53 | 160.69 | 120.27 | w12-B9 | 3587.56 | 1793.28 | 1195.19 | 896.14 |
| a2 | 561.14 | 280.07 | 186.38 | 139.54 | a12 | 3691.68 | 1845.34 | 1229.89 | 922.17 |
| w2 | 635.11 | 317.06 | 211.04 | 158.03 | w12 | 3722.62 | 1860.81 | 1240.21 | 929.91 |

(fortgesetzt)

|  | Ladungszustand | | | |  | Ladungszustand | | | |
|---|---|---|---|---|---|---|---|---|---|
| Typ | -1 | -2 | -3 | -4 | Typ | -1 | -2 | -3 | -4 |
| a3-B3 | 739.15 | 369.08 | 245.72 | 184.04 | a13-B13 | 3869.68 | 1934.34 | 1289.23 | 966.67 |
| w3-B18 | 813.11 | 406.06 | 270.37 | 202.53 | w13-B8 | 3900.62 | 1949.81 | 1299.54 | 974.41 |
| a3 | 850.19 | 424.60 | 282.73 | 211.80 | a13 | 4004.74 | 2001.87 | 1334.25 | 1000.44 |
| w3 | 964.16 | 481.58 | 320.72 | 240.29 | w13 | 4035.67 | 2017.34 | 1344.56 | 1008.17 |
| a4-B4 | 1028.19 | 513.60 | 342.06 | 256.30 | a14-B14 | 4182.74 | 2090.87 | 1393.58 | 1044.94 |
| w4-B17 | 1142.16 | 570.58 | 380.05 | 284.79 | w14-B7 | 4213.68 | 2106.34 | 1403.89 | 1052.67 |
| a4 | 1163.25 | 581.13 | 387.08 | 290.06 | a14 | 4308.78 | 2153.89 | 1435.59 | 1076.45 |
| w4 | 1268.21 | 633.61 | 422.07 | 316.30 | w14 | 4348.73 | 2173.87 | 1448.91 | 1086.43 |
| a5-B5 | 1341.25 | 670.13 | 446.42 | 334.56 | a15-B15 | 4486.79 | 2242.90 | 1494.93 | 1120.95 |
| w5-B16 | 1446.21 | 722.61 | 481.40 | 360.80 | w15-B6 | 4526.73 | 2262.87 | 1508.24 | 1130.93 |
| a5 | 1492.30 | 745.65 | 496.77 | 372.33 | a15 | 4612.83 | 2305.92 | 1536.94 | 1152.46 |
| w5 | 1557.25 | 778.13 | 518.42 | 388.56 | w15 | 4677.78 | 2338.39 | 1558.59 | 1168.70 |
| a6-B6 | 1670.30 | 834.65 | 556.10 | 416.83 | a16-B16 | 4790.83 | 2394.92 | 1596.28 | 1196.96 |
| w6-B15 | 1735.26 | 867.13 | 577.75 | 433.07 | w16-B5 | 4855.79 | 2427.40 | 1617.93 | 1213.20 |
| a6 | 1821.35 | 910.18 | 606.45 | 454.59 | a16 | 4901.87 | 2450.44 | 1633.29 | 1224.72 |
| w6 | 1861.30 | 930.15 | 619.77 | 464.58 | w16 | 5006.83 | 2502.92 | 1668.28 | 1250.96 |
| a7-B7 | 1999.35 | 999.18 | 665.78 | 499.09 | a17-B17 | 5079.88 | 2539.44 | 1692.63 | 1269.22 |
| w7-B14 | 2039.30 | 1019.15 | 679.10 | 509.08 | w17-B4 | 5184.84 | 2591.92 | 1727.61 | 1295.46 |
| a7 | 2134.41 | 1066.71 | 710.80 | 532.85 | a17 | 5205.92 | 2602.46 | 1734.64 | 1300.73 |
| w7 | 2165.34 | 1082.17 | 721.11 | 540.59 | w17 | 5319.89 | 2659.45 | 1772.63 | 1329.22 |
| a8-B8 | 2312.41 | 1155.71 | 770.14 | 577.35 | a18-B18 | 5383.92 | 2691.46 | 1793.97 | 1345.23 |
| w8-B13 | 2343.35 | 1171.18 | 780.45 | 585.09 | w18-B3 | 5497.89 | 2748.45 | 1831.96 | 1373.72 |
| a8 | 2447.47 | 1223.24 | 815.16 | 611.12 | a18 | 5534.97 | 2766.99 | 1844.32 | 1382.99 |
| w8 | 2478.40 | 1238.70 | 825.47 | 618.85 | w18 | 5608.94 | 2803.97 | 1868.98 | 1401.49 |
| a9-B9 | 2625.47 | 1312.24 | 874.49 | 655.62 | a19-B19 | 5712.98 | 2855.99 | 1903.66 | 1427.50 |
| w9-B12 | 2656.40 | 1327.70 | 884.80 | 663.35 | w19-B2 | 5786.94 | 2892.97 | 1928.31 | 1445.99 |
| a9 | 2760.52 | 1379.76 | 919.51 | 689.38 | a19 | 5864.02 | 2931.51 | 1954.01 | 1465.26 |
| w9 | 2767.45 | 1383.23 | 921.82 | 691.11 | w19 | 5922.00 | 2960.50 | 1973.33 | 1479.75 |
| a10-B10 | 2938.53 | 1468.77 | 978.84 | 733.88 | [M] | 6171.08 | 3085.04 | 2056.36 | 1542.02 |
| w10-B11 | 2945.45 | 1472.23 | 981.15 | 735.61 | | | | | |
| w10 | 3080.51 | 1539.76 | 1026.17 | 769.38 | | | | | |
| a10 | 3089.58 | 1544.29 | 1029.19 | 771.65 | | | | | |

**Patentansprüche**

1.  Verfahren zur Sequenzierung von Nukleinsäuren, wobei die für eine angenommene Sequenz berechneten Werte des CID-Spektrums mit dem gemessenen Spektrum der Nukleinsäure verglichen und der Grad der Übereinstimmung berechnet wird, **dadurch gekennzeichnet, dass**
    an jeder Stelle der angenommenen Sequenz jedes der vier möglichen Nukleotide A, U oder T, G, C eingesetzt und der Grad der Übereinstimmung mit dem gemessenen Spektrum jeweils neu berechnet wird und dass
    zur Berechnung der Übereinstimmung von berechnetem und gemessenem Spektrum ein Algorithmus verwendet wird, welcher auf der Feststellung beruht, ob an der berechneten Stelle im gemessenen Spektrum ein Messwert vorliegt oder nicht und dass ein größerer Grad von Übereinstimmung angenommen wird,
    wenn die Messwerte höhere Intensität (I) und geringere Abweichung ($\Delta$) vom exakten Wert für m/z aufweisen, und je größer die Zahl der Zuordnungen (K) und je geringer die Summe an nichtabgedeckten Positionen (M) ist.

2.  Verfahren nach Anspruch 1, **dadurch gekennzeichnet, dass** der Algorithmus zur Berechnung der Übereinstimmung durch Vergleich einer angenommenen Sequenz und des Spektrums unter der Voraussetzung, dass das Spektrum dieser Sequenz genau entspricht, festgesetzt wird.

**Claims**

1.  Method for sequencing nucleic acids in which the values of the CID spectrum calculated for an assumed sequence are compared with the measured spectrum of the nucleic acid and the degree of concordance is calculated, **characterized in that**
    each of the four possible nucleotides A, U or T, G, C is inserted at each position of the assumed sequence and the degree of concordance with the measured spectrum is recalculated in each case and that
    an algorithm is used to calculate the concordance of the calculated and measured spectrum which is based on a determination of whether a measured value is present or not at the calculated position in the measured spectrum and that a higher degree of concordance is assumed
    when the measured values have a higher intensity (I) and lower deviation ($\Delta$) from the exact value for m/z and the larger the number of allocations (K) and the lower the sum of non-covered positions (M).

2.  Method according to claim 1, **characterized in that** the algorithm for calculating the concordance is determined by comparing an assumed sequence and the spectrum provided that the spectrum exactly corresponds to this sequence.

**Revendications**

1.  Procédé de séquençage d'acides nucléiques, dans lequel les valeurs calculées pour une séquence supposée du spectre CID est comparée avec le spectre mesuré de l'acide nucléique et le degré de coïncidence est calculé, **caractérisé en ce que**
    à chaque position de la séquence supposée, chacun des quatre nucléotides possibles A, U ou T, G, C, est mis en oeuvre et le degré de coïncidence est calculé à nouveau et recalculé avec le spectre mesuré, et **en ce que**
    pour calculer la coïncidence du spectre calculé et mesuré, un algorithme est employé, qui se base sur la constatation qu'au niveau de la position calculée dans le spectre mesuré, une valeur mesurée existe ou non, et qu'un degré de coïncidence plus élevé est supposé,
    lorsque les valeurs mesurées présentent une intensité plus élevée (I) et une plus faible déviation ($\Delta$) de la valeur exacte pour m/z, et
    plus le nombre des associations (K) est grand et plus la somme de positions non couvertes (M) est petite.

2.  Procédé selon la revendication 1, **caractérisé en ce que** l'algorithme pour calculer la coïncidence par comparaison d'une séquence supposé et du spectre est fixé, avec la condition préalable que le spectre corresponde exactement à cette séquence.

(a) Vorläuferion

(b) Fragmentionen

(c) Fragmentionenspektrum

FIG. 1

**Eingabe:**

| Referenzsequenz<br>Ladungszustand des Vorläuferions | gemessenes MS/MS-Spektrum<br>(m/z, %Intensität) |

Schritt 1: Berechnung der m/z der
a-, a-B-, w-, w-B-Ionserien

berechnete Fragmentionen
(m/z der a-, a-B-, w-, w-B-Ionserien)

Schritt 2a: Vergleich der berechneten und
der gemessenen m/z-Werte

K ... Zahl der zugeordneten Fragmente
M ... Summe der nicht abgedeckten Positionen
für eine vorher festgesetzte maximale absolute
Massenabweichung $\Delta$

Schritt 2b:
Berechnung von K und M
für $\Delta$=0.2, 0.3, ... 0.8

Schritt 2c: Auswahl des optimalen $\Delta$-Wertes
Schritt 3: Berechnung des Übereinstimmungsfaktors

$$MF = 1/K \cdot \Sigma (100 \cdot \Delta/I\%)$$

Schritt 4: Berechnung des Koeffizienten b

$$b = MF/K \cdot 1,3$$

Schritt 5: Berechnung der Fitness FS
mit dem Koeffizienten c = 0,1

$$FS = MF - b \cdot K + 0,1 \cdot M$$

Schritt 6
a: Permutation der Referenzsequenz mittels Austausch einer Base
b: Berechnung der a-, a-B-, w-, w-B-Fragmentmassen
c: Vergleich der berechneten und experimentellen m/z-Werte
d: Berechnung der neuen Fitness
e: für eine Sequenz der Länge n ergeben sich
   3n Berechnungen der Fitness

**Ausgabe:**

| Position | Sequenz | FS(A) | FS(C) | FS(G) | FS(T) |
|---|---|---|---|---|---|
| 1 | T | 15,93 | 15,75 | 15,17 | -5.53 |
| 2 | G | 23,34 | 20,26 | -5,53 | 12.26 |
| 3 | G | 1,34 | 6,90 | -5,53 | -2.78 |
| 4 | C | 5,20 | -5,53 | 11,59 | -4.27 |

FIG. 2

FIG. 3

FIG. 4

FIG. 5

FIG. 6

FIG. 7

FIG. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US PS6017693 A, Yates

### In der Beschreibung aufgeführte Nicht-Patentliteratur

- **NI, J. ; POMERANTZ, S. C. ; ROZENSKI, J. ; ZHANG, Y. ; MCCLOSKEY J.A.** *Anal. Chem.,* 1996, vol. 68, 1989-99

SEQUENCE LISTING

<110> F.Hoffmann-La Roche AG

<120> Verfahren zur Sequenzierung von Nukleinsäuren

<130> 21385WO-Ra

<150> AT 1444/2001
<151> 2001-09-14

<160> 4

<170> PatentIn version 3.1

<210> 1
<211> 20
<212> DNA
<213> artificial

<400> 1
gacaggaaag actttctggc          20

<210> 2
<211> 20
<212> DNA
<213> artificial

<400> 2
gacaggaaag acattctggc          20

<210> 3
<211> 51
<212> DNA
<213> artificial

<400> 3
aaaccacatt ctgagcatac ccccaaaaaa tttcatgccg aagctgtggt c          51

<210> 4
<211> 80
<212> DNA
<213> artificial

<400> 4

```
ccccagtgct gcaatgatac cgcgagaccc acgctcaccg gctccagatt tatcagcaat      60

aaaccagcca gccggaaggg                                                   80
```